# EUROPEAN PATENT SPECIFICATION

(11) **EP 1 354 575 B1**
(45) Date of publication and mention of the grant of the patent: **17.12.2008**
(21) Application number: 02425232.2
(22) Date of filing: 15.04.2002
(51) Int. Cl.: A61F 13/15, A61F 13/551

(54) **Packaging for feminine hygiene pads**
Damenbindenverpackung
Emballage pour serviettes hygiéniques

(43) Date of publication of application: 22.10.2003
(73) Proprietor: Poligof S.r.l., 26854 Pieve Fissiraga (LO) (IT)
(72) Inventor: Buongiorno, Livio, 20090 Trezzano Sul Naviglio(Milano) (IT); Gatti, Giovanni, 26900 Lodi (IT)
(74) Representative: Petruzziello, Aldo

(56) References cited:
- EP-A- 0 880 954
- EP-A- 1 097 878
- PATENT ABSTRACTS OF JAPAN vol. 015, no. 436 (C-0882), 7 November 1991 (1991-11-07) & JP 03 184543 A (DAINIPPON PRINTING CO LTD), 12 August 1991 (1991-08-12)

## Description

The present invention refers to a packaging for a feminine hygiene pad or sanitary towel for women.

As is known, feminine hygiene pads comprise a body of absorbent material, able to absorb liquids, applied to a waterproof layer able to create a barrier to liquids. Adhesive strips are provided on an outer surface of the waterproof layer of the pad to allow the pad to be applied and held on the user's panties.

Feminine hygiene pads are generally packaged individually in packages made of protective film material, such as plastic material for example. To avoid having the adhesive strips adhere to the protective packaging, with the result of difficulty in unwrapping the pad and possible deterioration of said protective strips, strips of siliconed paper of the peel-off type are applied to the adhesive strips of the pad and serve to separate the adhesive strips of the pad from the packaging of plastic film. In this manner, once the pad has been unwrapped, the user can remove the peel-off strips of siliconed paper to apply the pad.

Such a package proves complex, uneconomical and awkward, due to the presence of the strips of siliconed paper to be applied to the adhesive strips. In fact the strips of siliconed paper represent an additional material in the package and application thereof represents an additional manufacturing process during packaging.

This drawback is overcome at least in part by packages known on the market as "single wrap film" which do not require the use of strips of siliconed paper to cover the adhesive strips on the pad. Said packages are made of films of polyolefin materials with or without the addition of "antiblocking" additives, such as silicas, which serve to make the material less sticky and improve the extrusion process.

The film of polyolefin materials is siliconed on the surface destined to face toward the inside of the package, by means of liquid silicone mineral oils. Consequently the siliconed layer comes into contact with the adhesive strips of the pad. As a result the adhesive strips of the pad, being in contact with the siliconed layer, do not adhere definitively to the package.

It is obvious that the production process of such a package proves complex and costly, above all because of the phase of depositing the liquid silicone mineral oils on the surface layer.

EP 0 880 954 discloses a packaging sheet for a sanitary napkin prepared by coating a release agent containing an epoxy functional polyorganosiloxane as the main component of the surface of the base material and then irradiating ultraviolet rays.

EP 1 097 878 discloses a web-like packaging material comprising non stick coatings consisting of polysiloxanes. The polysiloxane coatings are cured either termally or by radiation.

An objective of the present invention is to overcome the drawbacks of the prior art by providing a package for feminine hygiene pads that is practical, economical and easy to make.

Another objective of the present invention is to provide such a package for feminine hygiene pads that is practical for the user and at the same time able to ensure good standards of hygiene so as to preserve and protect the pad in it.

Another object of the present invention is to provide such a package for feminine hygiene pads that allows an extremely simple, fast and economical production process.

These objects are achieved in accordance with the invention with the characteristics listed in appended independent claim 1.

Advantageous embodiments of the invention are apparent from the dependent claims.

The package for feminine hygiene pads according to the invention comprises a functional layer, destined to face inward, in contact with the adhesive layer of the pad. Said functional layer comprises a thermoplastic resin containing a siloxane polymer. An ultra-high molecular weight (UHMW) siloxane polymer is preferably used, in a weight percentage in the range of 0.5% to 30%.

In this manner the functional layer has a surface with a low coefficient of friction, that is, with low adherence to the adhesive strips of the pad. As a result, the adhesive strips of the pad, which are in contact with the surface of the functional layer of the package, can be easily detached, without causing deterioration thereof.

The advantages of the package according to the invention are obvious in that it makes it possible to eliminate the additional manufacturing processes foreseen for packages of the prior art, such as deposition of the layer of silicone on the inner surface of the package, or application of the strips of siliconed paper to the adhesive strips of the pad.

Following various tests, the applicant has surprisingly discovered that for production of a package for feminine hygiene pads, a single-layer or multi-layer film of thermoplastic material can be used in which the functional layer, facing toward the inside of the package, comprises a siloxane polymer, **preferably** with ultrahigh molecular weight, such as polydimethylsiloxane, for example.

The presence of the siloxane polymer ensures a low coefficient of friction on the surface of the package in contact with the adhesive strips of the pad. In this manner the pad can be removed from the package, without the risk of its adhesive strips adhering to the surface of the package and being damaged. Said package according to the invention makes it possible to avoid the further process of silicone coating to form an anti-adhesive layer in contact with the adhesive strips of the pad.

The package according to the invention can be single-layered or multi-layered. The peculiar characteristic is represented by the fact that the functional layer, that is to say the layer of the package facing toward the inside, is made of thermoplastic material comprising one or more siloxane polymers.

Said siloxane polymer is supplied by means of masterbatches (MB), commonly available on the market, such as, for example, the masterbatches produced by Dow Corning^{™}. The siloxane masterbatch is supplied in solid granular form and generally comprises particles of ultrahigh molecular weight siloxane polymers, dispersed in various types of thermoplastic material, such as for example a polyolefin matrix. The percentage of particles of siloxane polymers in the masterbatch can vary from 1% to 80%, preferably from 25% to 50%. The particles of siloxane polymer generally have a mean grain size of about 5 micron, and a viscosity greater than 15 million (cSt).

The siloxane masterbatch thus obtained is then mixed with thermoplastic resins to be sent for extrusion. A siloxane masterbatch is put in said mixture for extrusion in a weight percentage that varies from 0.5% to 30%, preferably from 1% to 10%, even more preferably from 1.5% to 5%, with an optimal value of 3%.

Preferably polyethylenes (PE), such as low density polyethylene (LDPE), linear medium density polyethylene (LMDPE), linear low density polyethylene (LLDPE), and high density polyethylene (HDPE) can be used as thermoplastic resins for mixing with the masterbatch (MB) of siloxane. Ethylene copolymers such as ethyl vinyl acetate (EVA), ethyl meta methacrylate (EMMA), ethyl metacrylate (EMA), ethyl butyl acrylate (EBA) or metallocene copolymers can also be used. Thermoplastic resins such as polypropylene (PP), polyoxymethylene (POM), styrene and acrylonitrile copolymer (SAN) and polyethylene terephthalate (PET) can also be used.

It must be considered that during extrusion, the high molecular weight siloxane, once dispersed in the olefin polymer matrix, is trapped in said matrix and does not tend to migrate toward the surface. In this manner said extruded siloxane polymer eliminates the booming effect, and likewise the migration of fluids and other organic additives toward the surface, as happens in silicone based materials according to the prior art.

The main advantages of said siloxane polymers are:
- an increase in the yield and a reduction in energy consumption during extrusion,
- an improvement in the internal lubrication and flow of the thermoplastic material,
- an improvement in filling of the mould and in the aesthetic shape obtained,
- a reduction in the coefficient of friction of the polymer obtained without impairing the subsequent finishing operations such as printing, coating and painting.

The total thickness of the package can be from 10 to 70 microns, preferably about 30 microns. If a multilayered film is used, the thickness of the siloxane-based functional layer can be from 3 to 50 microns, preferably about 10 microns.

In the case of a package consisting of a multilayered film, the layer or layers supporting the functional layer can be made of thermoplastic materials, polyoleofin copolymers preferably being used. In particular, the supporting layers can comprise polyoleofin copolymers such as polyethylenes (PE), polypropylenes (PP) and metallocenes. Low density polyethylene (LDPE) and linear low density polyethylene (LLDPE) is preferably employed.

The thermoplastic materials employed for the production of the package for a hygiene or sanitary pad according to the invention can have chemical additive compounds added, such as antiblocking agents, slip agents, antioxidants and the like, to ensure good processability during extrusion. In particular, the siloxane-based functional layer can have a percentage of about 5% of antiblocking agent and a percentage of about 3% of slip agent.

The film of plastic material destined to form the package according to the invention can be produced by bubble extrusion or by flat extrusion, then wound into reels by means of winding systems. The material thus produced can undergo the subsequent manufacturing processes such as printing, sealing and the like to obtain the package for hygiene pads according to the invention.

It must be considered that the liquid silicone mineral oils employed for the siliconed coating of the packages according to the prior art cannot be mixed with polymer-based thermoplastic materials to be subsequently extruded as film since such an extruded film with silicone oils would present problems.

In fact, the silicone oil tends to migrate onto the surface layer of the film, creating a blooming effect, and tends to contaminate the surface, thus preventing sealing and printing of the film. Moreover, this surface migration of the silicone oil makes the film too slippery and does not allow some treatments of the film during the production and transformation of the film to be put on the market.

Instead the applicant, after various studies and experimental tests, has surprisingly discovered that a mixture - obtained by mixing a siloxane polymer with thermoplastic material - could be extruded and the film thus obtained had a low coefficient of friction, suitable not to adhere to the adhesive strips of the hygiene pad and at the same time said film did not give problems of surface migration of the silicone and therefore could undergo subsequent production processes, such as printing, painting, sealing and the like.

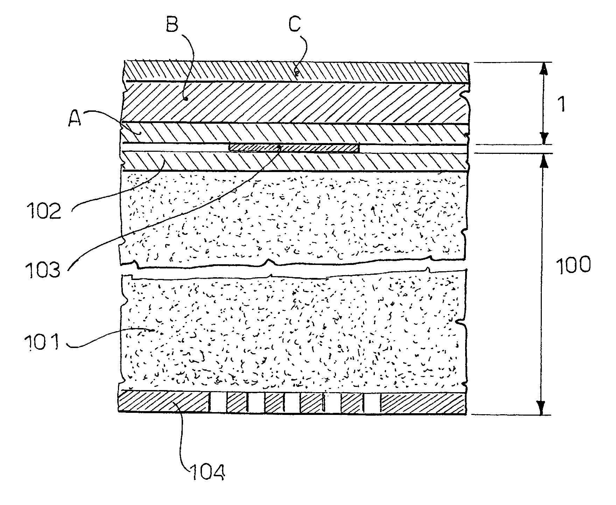

Further characteristics of the invention will be made clearer by the detailed description that follows, referring to a purely exemplary and therefore non limiting embodiment, illustrated in the appended drawing, which shows an enlarged, broken off sectional view, illustrating a package according to the invention, applied to a feminine hygiene pad.

In the drawing a hygiene pad, indicated as a whole with reference numeral 100, contained in a package according to the invention, is shown partially and denoted as a whole with reference numeral 1.

The hygiene pad comprises a body of absorbent material 101, interposed between a layer of microperforated material 104, on one side, and a layer of waterproof material 102, on the other side, whereon adhesive strips 103 are disposed.

The package 1 consists of a multilayer film comprising three layers of thermoplastic material A, B, C. A denotes the functional layer facing toward the inside of the package in contact with the adhesive strips 103 of the hygiene pad. B denotes an intermediate layer and C denotes the layer facing toward the outside of the package.

Hereunder, with reference to the drawing and by way of example, some exemplary embodiments of a package for hygiene pads according to the invention, obtained from a three-layered film of plastic material, are shown. In the examples that follow MB SILOXANE 1 indicates a masterbatch of siloxane with a percentage of siloxane polymer of about 25%, whereas MB SILOXANE 2 indicates a masterbatch of siloxane with a percentage of siloxane polymer of about 50%.

| Exemple 1 | | | | | | |
|---|---|---|---|---|---|---|
| LAYER | A INNER | % | B MIDDLE | % | C OUTER | % |
| MATERIALS | LDPE | 92 | LDPE | 92 | LLDPE | 100 |
| | SILOXANE MB 1 | 8 | WITHE MB | 8 | | |
| THICKNESS (µ) Total = 35 µ | 6,3 | | 22,4 | | 6,3 | |
| % WEIGHT | 0,18 | | 0,64 | | 0,18 | |

| Exemple 2 | | | | | | |
|---|---|---|---|---|---|---|
| LAYER | A INNER | % | B MIDDLE | % | C OUTER | % |
| MATERIALS | LDPE | 85 | LDPE | 92 | LLDPE | 100 |
| | SILOXANE MB 1 | 6 | WITHE MB | 8 | | |
| | ANTIBLOCK MB 1 | 5 | | | | |
| | SLIP MB | 3 | | | | |
| | NTIBLOCKMB A 2 | 1 | | | | |
| THICKNESS (µ) Total = 35 µ | 6,3 | | 22,4 | | 6,3 | |
| % WEIGHT | 0,18 | | 0,64 | | 0,18 | |

| Exemple 3 | | | | | | |
|---|---|---|---|---|---|---|
| LAYER | A INNER | % | B MIDDLE | % | C OUTER | % |
| MATERIALS | LDPE | 84 | LDPE | 92 | LLDPE | 100 |
| | SILOXANE MB 1 | 6 | WITHE MB | 8 | | |
| | MINERAL MB | 10 | | | | |
| THICKNESS (µ) Total = 35 µ | 6,3 | | 22,4 | | 6,3 | |
| % WEIGHT | 0,18 | | 0,64 | | 0,18 | |

| Exemple 4 | | | | | | |
|---|---|---|---|---|---|---|
| LAYER | A INNER | % | B MIDDLE | % | C OUTER | % |
| MATERIALS | LDPE | 95 | LDPE | 92 | LLDPE | 100 |
| | SILOXANE MB 1 | 5 | WITHE MB | 8 | | |
| THICKNESS (µ) Total = 35 µ | 6,3 | | 22,4 | | 6,3 | |
| % WEIGHT | 0,18 | | 0,64 | | 0,18 | |

| Exemple 5 | | | | | | |
|---|---|---|---|---|---|---|
| LAYER | A INNER | % | B MIDDLE | % | C OUTER | % |
| MATERIALS | LDPE | 92 | LDPE | 92 | LLDPE | 100 |
| | SILOXANE MB 1 | 8 | WITHE MB | 8 | | |
| THICKNESS (µ) Total = 35 µ | 6,3 | | 22,4 | | 6,3 | |
| % WEIGHT | 0,18 | | 0,64 | | 0,18 | |

| Exemple 6 | | | | | | |
|---|---|---|---|---|---|---|
| LAYER | A INNER | % | B MIDDLE | % | C OUTER | % |
| MATERIALS | LDPE | 70 | LDPE | 92 | LLDPE | 100 |
| | SILOXANE MB 2 | 30 | WITHE MB | 8 | | |
| THICKNESS (µ) Total = 35 µ | 6,3 | | 22,4 | | 6,3 | |
| % WEIGHT | 0,18 | | 0,64 | | 0,18 | |

| Exemple 7 | | | | | | |
|---|---|---|---|---|---|---|
| LAYER | A INNER | % | B MIDDLE | % | C OUTER | % |
| MATERIALS | LDPE | 97 | LDPE | 92 | LLDPE | 100 |
| | SILOXANE MB 2 | 3 | WITHE MB | 8 | | |
| THICKNESS (µ) Total = 35 µ | 6,3 | | 22,4 | | 6,3 | |
| % WEIGHT | 0,18 | | 0,64 | | 0,18 | |

| Exemple 8 | | | | | | |
|---|---|---|---|---|---|---|
| LAYER | A INNER | % | B MIDDLE | % | C OUTER | % |
| MATERIALS | LDPE | 70 | LDPE | 92 | LLDPE | 100 |
| | SILOXANE MB 2 | 30 | WITHE MB | 8 | | |
| THICKNESS (µ) Total = 30 µ | 5,5 | | 19 | | 5,5 | |
| % WEIGHT | 0,18 | | 0,63 | | 0,18 | |

| Exemple 9 | | | | | | |
|---|---|---|---|---|---|---|
| LAYER | A INNER | % | B MIDDLE | % | C OUTER | % |
| MATERIALS | LDPE | 97 | LDPE | 92 | LLDPE | 100 |
| | SILOXANE MB 1 | 3 | WITHE MB | 8 | | |
| THICKNESS (µ) Total = 30 µ | 5,5 | | 19 | | 5,5 | |
| % WEIGHT | 0,18 | | 0,64 | | 0,18 | |

As can be noted from the examples, the internal functional layer A advantageously comprises a siloxane polymer obtained by mixing LDPE (low density polyethylene) with a siloxane masterbatch, the central layer B comprises LDPE (low density polyethylene) with white masterbatch added and the outer layer C comprises LLDPE (linear low density polyethylene).

In example 2 in particular, the siloxane polymer of the functional layer A has antiblock and slip agent masterbatch added, whereas in example 3 mineral masterbatch is added.

Numerous variations and modifications of detail within the reach of a person skilled in the art can be made to the present embodiment of the invention without thereby departing from the scope of the invention expressed in the appended claims.

## Claims

1. A package (1) for a feminine hygiene pad (100), the feminine hygiene pad comprising a body of absorbent material (101) interposed between a layer of microperforated material (104), on one side, and a layer of waterproof material (102), on the other side, on which adhesive strips (103) are disposed, wherein said package (1) comprises a functional layer (A), facing toward the inside of the package, said functional layer (A) consisting of thermoplastic material comprising a siloxane polymer with a low coefficient of friction toward said adhesive strips (103) of the hygiene pad (100), to allow easy detachment of the hygiene pad (100) from the package (1), without damage to the relative adhesive strips (103),
said functional layer (A) is obtainable by extrusion of a siloxane polymer masterbatch (MB) mixed with thermoplastic resins.

2. A package according to claim 1, **characterised in that** said functional layer (A) comprises an ultrahigh molecular weight (UHMW) siloxane polymer, such as polydimethyl siloxane.

3. A package according to claim **1 or 2, characterised in that** said siloxane masterbatch (MB) comprises particles of siloxane polymer dispersed in a matrix of olefinic polymers.

4. A package according to claim 3, **characterised in that** said particles of siloxane polymer have a mean grain size of about 5 micron.

5. A package according to claim 3 or 4, **characterised in that** said particles of siloxane polymer have a viscosity greater than 15 million (cSt).

6. A package according to any one of claims 3 to 5, **characterised in that** said siloxane masterbatch (MB) contains a weight percentage of siloxane polymer particles between 1% and 80%, preferably between 25% and 50%.

7. A package according to any one of claims 2 to 6, **characterised in that** the functional layer (A) contains a weight percentage of siloxane masterbatch (MB) between 0.5% and 30%, preferably between 1% and 10%.

8. A package according to any one of claims 2 to 7, **characterised in that** said thermoplastic resins of the functional layer (A) comprise polyolefinic resins such as polyethylene (PE), low density polyethylene (LDPE), linear medium density polyethylene (LMPDE), linear low density polyethylene (LLPDE) and/or high density polyethylene (HDPE).

9. A package according to any one of claims 2 to 8, **characterised in that** said thermoplastic resins of the functional layer (A) comprise ethylene copolymers such as ethyl-vinyl-acetate (EVA), ethyl-methyl-meta-acrylate (EMMA), ethyl-meta-acrylate (MMA) and/or ethyl-butane-acrylate (EBA) or mixtures thereof.

10. A package according to any one of the preceding claims, **characterised in that t**he thermoplastic material of said functional layer (A) comprises additives such as antiblocking and/or slip agents.

11. A package according to any one of the preceding claims, **characterised in that** it is obtained from a multilayered film.

12. A package according to claim 11, **characterised in that** the total thickness of the package is between 10 microns and 70 microns, preferably about 30 microns, and the thickness of the functional layer (A) is between 3 and 50 microns, preferably about 10 microns.

13. A package according to claim 11 or 12, **characterised in that** it comprises further layers (B, C) to support the functional layer (A) of the package, consisting of polyolefin copolymers such as low density polyethylene (LDPE) and/or linear low density polyethylene (LLDPE).

## Patentansprüche

1. Verpackung (1) für eine Damenbinde (100), wobei die Damenbinde einen Körper aus absorbierendem Material (101) aufweist, der zwischen eine Schicht aus mikroperforiertem Material (104) auf einer Seite und eine Schicht aus wasserdichtem Material (102) auf der anderen Seite eingefügt ist, auf der Klebestreifen (103) angeordnet sind, wobei die Verpackung (1) eine funktionale Schicht (A) aufweist, die zur Innenseite der Packung zeigt, wobei die funktionale Schicht (A) aus Thermoplastmaterial besteht, das einen Siloxanpolymer mit niedrigem Reibungskoeffizienten zu den Klebestreifen (103) der Damenbinde (100) aufweist, um ein leichtes Ablösen der Damenbinde (100) von der Verpackung (1) ohne Schäden an den Klebestreifen (103) zu erlauben, wobei die funktionale Schicht (A) durch Extrusion aus einer Siloxanpolymer-Vormischung (MB), die mit Thermoplastharzen gemischt ist, erzielt werden kann.

2. Verpackung nach Anspruch 1, **dadurch gekennzeichnet, dass** die funktionale Schicht (A) ein ultrahochmolekulares (UHMW) Siloxanpolymer aufweist, wie zum Beispiel Polydimethylsiloxan.

3. Verpackung nach Anspruch 1 oder 2, **dadurch gekennzeichnet, dass** die Siloxan-Vormischung (MB) Partikel aus Siloxanpolymer in einer Matrix aus olefinischen Polymeren dispergiert aufweist.

4. Verpackung nach Anspruch 3, **dadurch gekennzeichnet, dass** die Siloxanpolymerpartikel eine Hauptkomgröße von etwa 5 Mikrometer haben.

5. Verpackung nach Anspruch 3 oder 4, **dadurch gekennzeichnet, dass** die Siloxanpolymerpartikel eine Viskosität größer als 15 Millionen (cSt) haben.

6. Verpackung nach einem der Ansprüche 3 bis 5, **dadurch gekennzeichnet, dass** die Siloxan-Vormischung (MB) einen Gewichtsprozentsatz an Siloxanpolymerpartikeln zwischen 1 % und 80 %, vorzugsweise zwischen 25 % und 50 % enthält.

7. Verpackung nach einem der Ansprüche 2 bis 6, **dadurch gekennzeichnet, dass** die funktionale Schicht (A) einen Gewichtsprozentsatz an Siloxan-Vormischung (MB) zwischen 0,5 % und 30 %, vorzugsweise zwischen 1 % und 10 % aufweist.

8. Verpackung nach einem der Ansprüche 2 bis 7, **dadurch gekennzeichnet, dass** die Thermoplastharze der funktionalen Schicht (A) Polyolefinharze aufweisen, wie zum Beispiel Polyethylen (PE), niedrigdichtes Polyethylen (LDPE), lineares mitteldichtes Polyethylen (LMPDE), lineares niedrigdichtes Polyethylen (LLPDE) und/oder hochdichtes Polyethylen (HDPE).

9. Verpackung nach einem der Ansprüche 2 bis 8, **dadurch gekennzeichnet, dass** die Thermoplastharze der funktionalen Schicht (A) Ethylencopolymere enthalten, wie zum Beispiel Ethyl-Vinyl-Acetat (EVA), Ethyl-Methyl-Meta-Acrylat (EMMA), Ethyl-Meta-Acrylat (MMA) und/oder Ethyl-Butan-Acrylat (EBA) oder Gemische davon.

10. Verpackung nach einem der vorhergehenden Ansprüche, **dadurch gekennzeichnet, dass** das Thermoplastmaterial der Funktionsschicht (A) Zusatzstoffe, wie zum Beispiel Antiblockingmittel und/oder Gleitmittel aufweist.

11. Verpackung nach einem der vorhergehenden Ansprüche, **dadurch gekennzeichnet, dass** sie aus einer vielschichtigen Folie erzielt wird.

12. Verpackung nach Anspruch 11, **dadurch gekennzeichnet, dass** die Gesamtstärke der Verpackung zwischen 10 Mikrometer und 70 Mikrometer, vorzugsweise etwa 30 Mikrometer liegt, und dass die Stärke der funktionalen Schicht (A) zwischen 3 und 50 Mikrometer, vorzugsweise etwa 10 Mikrometer liegt.

13. Verpackung nach Anspruch 11 oder 12, **dadurch gekennzeichnet, dass** sie ferner Schichten (B, C) aufweist, um die funktionale Schicht (A) der Packung zu tragen, die aus Polyolefincopolymeren bestehen, wie zum Beispiel niedrigdichtem Polyethylen (LDPE) und/oder linearem niedrigdichtem Polyethylen (LLDPE).

## Revendications

1. Emballage (1) pour serviette hygiénique (100), la serviette hygiénique comportant un corps de matière absorbante (101) intercalé entre une couche de matière microperforée (104) d'un côté et une couche de matière imperméable (102) de l'autre côté, sur lesquelles des bandes adhésives (103) sont disposées, ledit emballage (1) comportant une couche fonctionnelle (A) tournée vers l'intérieur de l'emballage, ladite couche fonctionnelle (A) étant composée de matière thermoplastique contenant un polymère de siloxane ayant un faible coefficient de friction par rapport auxdites bandes adhésives (103) de la serviette hygiénique (100) afin de permettre un détachement facile de la serviette hygiénique (100) de l'emballage (1) sans endommager les bandes adhésives relatives (103), ladite couche fonctionnelle (A) pouvant être obtenue par extrusion d'un mélange monté de polymère de siloxane (MB) mélangé à des résines thermoplastiques.

2. Emballage selon la revendication 1, **caractérisé en ce que** ladite couche fonctionnelle (A) contient un polymère de siloxane à poids moléculaire ultra-élevé (UHMW) comme un polydiméthyle siloxane.

3. Emballage selon la revendication 1 ou 2, **caractérisé en ce que** ledit mélange monté de siloxane (MB) contient des particules de polymère de siloxane dispersées dans une matrice de polymères oléfiniques.

4. Emballage selon la revendication 3, **caractérisé en ce que** lesdites particules de polymère de siloxane ont une taille moyenne de grains d'environ 5 microns.

5. Emballage selon la revendication 3 ou 4, **caractérisé en ce que** lesdites particules de polymère de siloxane ont une viscosité supérieure à 15 millions (cSt).

6. Emballage selon l'une quelconque des revendications 3 à 5, **caractérisé en ce que** ledit mélange monté de polymère de siloxane (MB) contient un pourcentage en poids de particules de polymère de siloxane allant de 1 % à 80 %, de préférence 25 % à 50 %.

7. Emballage selon l'une quelconque des revendications 2 à 6, **caractérisé en ce que** la couche fonctionnelle (A) contient un pourcentage en poids de mélange monté de siloxane (MB) allant de 0,5 % à 30 %, de préférence 1 % à 10 %.

8. Emballage selon l'une quelconque des revendications 2 à 7, **caractérisé en ce que** lesdites résines thermoplastiques de la couche fonctionnelle (A) contiennent des résines polyoléfiniques comme du polyéthylène (PE), du polyéthylène à basse densité (LDPE), du polyéthylène à densité moyenne linéaire (LMPDE), du polyéthylène à basse densité linéaire (LLPDE) et/ou du polyéthylène à haute densité (HDPE).

9. Emballage selon l'une quelconque des revendications 2 à 8, **caractérisé en ce que** lesdites résines thermoplastiques de la couche fonctionnelle (A) contiennent des copolymères d'éthylène comme de l'éthyle-vinyle-acétate (EVA), de l'éthyle-méthyle-méta-acrylate (EMMA), de l'éthyle-méta-acrylate (MMA) et/ou de l'éthyle-butane-acrylate (EBA) ou leurs mélanges.

10. Emballage selon l'une quelconque des revendications précédentes, **caractérisé en ce que** la matière thermoplastique de ladite couche fonctionnelle (A) contient des additifs comme des agents antibloquants et/ou de glissement.

11. Emballage selon l'une quelconque des précédentes, **caractérisé en ce qu'**il est obtenu à partir d'un film multicouche.

12. Emballage selon la revendication 11, **caractérisé en ce que** l'épaisseur totale de l'emballage est située entre 10 et 70 microns, de préférence à environ 30 microns, et que l'épaisseur totale de la couche fonctionnelle (A) est située entre 3 et 50 microns, de préférence à environ 10 microns.

13. Emballage selon la revendication 11 ou 12, **caractérisé en ce qu'**il comprend d'autres couches (B, C) destinées à supporter la couche fonctionnelle (A) de l'emballage et composées de copolymères de polyoléfine comme du polyéthylène à basse densité (LPDE) et/ou du polyéthylène à basse densité linéaire (LLPDE).
